# EUROPEAN PATENT SPECIFICATION

(11) **EP 4 609 015 B1**
(45) Date of publication and mention of the grant of the patent: **06.05.2026**
(21) Application number: 23809968.3
(22) Date of filing: 27.10.2023
(51) Int. Cl.: C25B 3/07, C25B 3/25, C25B 11/046, C25B 11/089

(54) **METHOD OF ELECTROCHEMICAL CONVERSION OF AQUEOUS SOLUTIONS OF CARBONATES, BICARBONATES, CO2, C2-C5 ACIDS, SALTS OF C2-C5 ACIDS AND MIXTURES THEREOF**
VERFAHREN ZUR ELEKTROCHEMISCHEN UMSETZUNG WÄSSRIGER LÖSUNGEN VON CARBONATEN, BIKARBONATEN, CO2-C5-SÄUREN, SALZEN VON C2-C5-SÄUREN UND MISCHUNGEN DAVON
PROCÉDÉ DE CONVERSION ÉLECTROCHIMIQUE DE SOLUTIONS AQUEUSES DE CARBONATES, BICARBONATES, CO

(30) Priority: 29.10.2022 CZ 20220447
(43) Date of publication of application: 03.09.2025
(73) Proprietor: Ustav Chemickych Procesu AV CR, V.V.I., 165 00 Praha 6 (CZ)
(72) Inventor: DYTRYCH, Pavel, 14900 Praha 4 (CZ); FAJGAR, Radek, 16000 Praha 6 (CZ); DRINEK, Vladislav, 16900 Praha 6 (CZ); KOSTEJN, Martin, 18100 Praha 8 (CZ); JANDOVA, Vera, 10900 Praha 10 (CZ)
(74) Representative: Harber IP s.r.o.
(86) International application number: PCT/CZ2023/050071
(87) International publication number: WO 2024/088453

(56) References cited:
- WO-A1-2014/149224
- CA-A1- 2 864 611
- US-A1- 2003 220 514
- SHANWEN WANG: "Electrochemical Reduction of CO 2 to Alcohols: Current Understanding, Progress, and Challenges", ADVANCED ENERGY AND SUSTAINABILITY RESEARCH, vol. 3, no. 1, 23 January 2022 (2022-01-23), XP093154043, ISSN: 2699-9412, Retrieved from the Internet <URL:https://onlinelibrary.wiley.com/doi/full-xml/10.1002/aesr.202100131> [retrieved on 20240419], DOI: 10.1002/aesr.202100131
- R. KORTLEVER: "Electrochemical carbon dioxide and bicarbonate reduction on copper in weakly alkaline media", JOURNAL OF SOLID STATE ELECTROCHEMISTRY, vol. 17, no. 7, 9 May 2013 (2013-05-09), DE, pages 1843 - 1849, XP093154039, ISSN: 1432-8488, Retrieved from the Internet <URL:https://link.springer.com/content/pdf/10.1007/s10008-013-2100-9.pdf> [retrieved on 20240419], DOI: 10.1007/s10008-013-2100-9

## Description

### Field of Art

The present invention relates to a method of electrochemically converting (reducing) aqueous solutions of alkali metal carbonates, ammonium carbonates, alkali metal bicarbonates, ammonium bicarbonate, CO₂, C2-C5 carboxylic acids, salts of C2-C5 carboxylic acids with alkali metals or ammonium, and mixtures thereof, or mixtures thereof with ammonia, to form alcohols and/or salts of carboxylic acids.

### Background Art

When a preparation of ethanol, methanol, propan-1-ol, isopropanol as a product of CO₂ reduction is required in practice, the selection of the electrocatalytic system is problematic due to the short lifetime of the cathode material, low selectivity for ethanol and low yields, i.e., low conversion of CO₂ in the form of carbonates, bicarbonates and free CO₂ in a carbonate solution.

Only electrocatalytic systems containing metallic copper are available Shanwen Wang, Electrochemical Reduction of CO2 to Alcohols: Current Understanding, Progress, and Challenges, Advanced Energy and Sustainability Research, 23 January 2022, Vol. 3, No. 1, DOI: 10.1002/aesr.202100131, R. Kortlever, Electrochemical carbon dioxide and bicarbonate reduction on copper in weakly alkaline media, Journal of Solid State Electrochemistry, 9 May 2013, Vol. 17, No. 7, pages 1843-1849, DOI: 10.1007/s10008-013-2100-9, CA 2864611). Copper is the only known material to allow the formation of C₂+ products by direct reduction of CO₂ in the form of carbonates, bicarbonates and free CO₂ in carbonate solution, but it degrades after a short time (< 100 h) and the selectivity to ethanol and other products is minimal at the expense of hydrogen evolution.

The present invention aims to overcome the drawbacks of the prior art.

### Disclosure of the Invention

The present invention provides a method for electrochemically converting a starting solution, selected from aqueous solutions of alkali metal carbonate, ammonium carbonate, alkali metal bicarbonate, ammonium bicarbonate, CO₂, a C2-C5 carboxylic acid, a C2-C5 carboxylic acid salt with an alkali metal or ammonium, or mixtures thereof, or mixtures thereof with ammonia, to form alcohols and/or carboxylic acid salts. The method is carried out in an electrochemical reactor in which the cathode is a catalyst comprising copper silicide, copper germanide and/or mixed copper germanide/silicide.

Preferably, in the electrochemical reactor, the cathode compartment and the anode compartment are separated by a proton exchange membrane. The selectivity of the reaction is thus significantly increased. The aqueous solution of alkali metal carbonate, ammonium carbonate, alkali metal bicarbonate, ammonium bicarbonate, CO₂, a C2-C5 carboxylic acid, a C2-C5 carboxylic acid salt with an alkali metal, or a mixture thereof, or a mixture thereof with ammonia, is used as the electrolyte in the electrochemical reactor. By the action of the catalyst cathode and by the passage of electric current, the conversions, in particular the conversion of carbonates, bicarbonates and CO₂ to alcohols and/or salts of C1-C3 carboxylic acids, and/or the conversion of salts of carboxylic acids to salts of higher carboxylic acids, take place.

In one preferred embodiment of the invention, the electrochemical conversion (reduction) process is carried out by submitting the starting electrolyte (starting solution), selected from aqueous solutions of alkali metal carbonate, ammonium carbonate, alkali metal bicarbonate, ammonium bicarbonate, CO₂ or a mixture thereof, or a mixture thereof with ammonia, to electrochemical reaction, preferably using copper silicide and/or mixed copper germanide/silicide cathode, and the reaction produces alcohols and salts of carboxylic acids. Typical products are methanol, ethanol, acetate, propionate, valerate, lactate, hydrogen in the cathode compartment, and oxygen in the anode compartment.

In this embodiment, the equilibrium of the reaction products can be influenced to some extent by the pH of the starting electrolyte. In the pH range of 6.7 to 9 (preferably 6.7 to 8.5), more alcohols (C1-C3 alcohols, in particular ethanol) are formed, and at pH higher than 9 (preferably 9.5 to 13.5), more C1-C3 salts of carboxylic acids (in particular acetate) are formed.

The starting electrolyte may be prepared by dissolving alkali metal carbonate, ammonium carbonate, alkali metal bicarbonate, ammonium bicarbonate or ammonia in water, thus producing basic solutions. Saturating such a solution with carbon dioxide or saturating water with carbon dioxide decreases the pH.

In another preferred embodiment, the electrochemical conversion (reduction) process is carried out by submitting the starting electrolyte (starting solution), which is an aqueous solution of an alkali metal or ammonium salt of C2-C5 carboxylic acid, to electrochemical reaction, preferably using copper germanide and/or mixed copper germanide/silicide cathode, and the reaction produces alcohols and carboxylic acid salts having a higher number of carbon atoms than the initial carboxylic acid. Depending on the reaction time, carboxylic acid salts with a number of carbon atoms of up to 10 can be prepared.

The reaction product of the reaction according to the preceding advantageous embodiment may be the starting electrolyte for this reaction, wherein this reaction increases the number of carbon atoms of the carboxylic acids in the reaction mixture.

In one preferred embodiment, the electrochemical conversion (reduction) process is carried out by submitting the starting electrolyte (starting solution), which is an aqueous solution of a C2-C5 carboxylic acid, to electrochemical reaction, preferably using copper silicide and/or mixed copper germanide/silicide cathode, and the reaction produces alcohol which may react in situ with the carboxylic acid to form an ester.

In yet another preferred embodiment, the electrochemical conversion (reduction) process is carried out by submitting the starting electrolyte (starting solution), selected from aqueous solutions of alkali metal carbonate, ammonium carbonate, alkali metal bicarbonate, ammonium bicarbonate, CO₂ or a mixture thereof, or a mixture thereof with ammonia, optionally further containing a C2-C5 carboxylic acid salt, to electrochemical reaction, preferably using a cathode made of a mixture of copper silicide and copper germanide and/or mixed copper germanide/silicide cathode, wherein salts of lower carboxylic acids, typically formate and acetate, are first formed, followed by a second in situ reaction of salts of lower carboxylic acids (in particular formates, acetates and propionates) to salts of carboxylic acids with a higher number of carbon atoms.

Within the framework of the present invention, it has been found that it is especially advantageous when the starting electrolyte contains ammonium cations. Ammonium cations significantly increase the yield of the electrochemical reaction and allow to increase current densities. A minor addition of ammonium ions, e.g. at least 1 mol. %, relative to the amount of carbonates, bicarbonates and/or carboxylic acid salts, is capable of bringing about this effect. More preferably, at least 10 mol. %, even more preferably at least 20 mol. % of ammonium ions, relative to the amount of carbonates, bicarbonates and/or carboxylic acid salts. Ammonium cations may be in the form of any water-soluble inorganic salt, including ammonium carbonate, ammonium bicarbonate, ammonium dihydrogenphosphate, ammonium hydrogenphosphate, ammonium phosphate, ammonium borate, ammonium tetraborate tetrahydrate (preferably in a mixture with boric acid), ammonium halogenide.

A particularly preferred embodiment of ammonium cations is ammonium carbonate, because it allows to achieve very high current densities. For example, saturated aqueous solution of ammonium carbonate, preferably with addition of ammonia, allows to achieve current densities around 1500 mA/cm².

In this disclosure, alkali metals are sodium, potassium, rubidium and caesium. The reactivity of alkali metal salts increases with increasing atomic number, but so does the price of these raw materials. Potassium has the best ratio of reactivity to price among the alkali metals. The highest reaction rates and selectivities were found in the combination of potassium and ammonium salts.

Copper silicides and copper germanides are intermetallic compounds that are known and commercially available. Methods for their preparation are also known.

A copper silicide is a compound with the formula CuₐSi, wherein a is in the range of 0.2 to 5 (i.e., typically ranging from Cu₅Si to CuSi₅). It can also be a mixture of silicides.

A copper germanide is a compound of the formula Cu_{b}Ge, wherein b is in the range of 0.2 to 5 (i.e. typically ranging from Cu₅Ge to CuGe₅). It can also be a mixture of germanides.

The mixed copper germanide/silicide is a compound of formula Cu_{c}Si_{d}Geₑ, wherein c is in the range of 1 to 5, d is in the range of 1 to 5, and e is in the range of 1 to 5. Preferably, the sum of d and e is in the range of 1 to 5.

The anode is preferably a platinum or stainless-steel electrode.

The input voltage may typically be in the range of 1.5 to 5 V, and the current densities may typically be in the range of 5 to 1500 mA/cm².

The reactions may be in a batch design or in a continuous (flow) design, as desired or as required.

The starting electrolyte, which is a solution selected from the solutions of alkali metal carbonate, ammonium carbonate, alkali metal bicarbonate, ammonium bicarbonate, CO₂ or a mixture thereof, or a mixture thereof with ammonia, can be prepared, for example, by first preparing a saturated alkali metal hydroxide (aqueous) solution which is then saturated with a gas containing CO₂, NH₃, preferably to a pH of not lower than 6.7. The CO₂-containing gas may be, for example, air, pure CO₂, flue gas, pyrolysis gas, fermenter gas, biogas, gas from other biochemical or synthetic production or from combustion, etc. Solutions containing a mixture of carbonates, bicarbonates and CO₂ and having a desired pH can then be prepared by dilution with water.

The starting electrolyte, which is a solution of an alkali metal salt of C2-C5 carboxylic acid or ammonium salt of C2-C5 carboxylic acid, can be prepared, for example, by first preparing a solution of the corresponding acid to which a saturated solution of alkali metal hydroxide or ammonium hydroxide is added. Solutions containing the salt in question at the desired concentration can then be prepared by dilution with water. The carboxylic acid may, for example, be a by-product of biochemical production. Incomplete neutralization of the acid is not suitable. A visible sign of sufficient/complete neutralization is transparency of the outflowing produced electrolyte, which can be monitored spectrally or by measuring parameters such as turbidity.

The method according to the present invention has a high lifetime of the cathode material of more than 600 hours, even at current densities higher than 1 A/cm². The reaction exhibits a higher selectivity towards the desired products than that of electrochemical reactions known in the prior art, and the selectivity can furthermore be controlled as a function of pH.

### Brief Description of Drawings

Fig. 1 schematically illustrates an example of an apparatus for preparing a starting electrolyte in the form of an aqueous solution of carbonates, bicarbonates and/or CO₂.
Fig. 2 schematically illustrates an example of an apparatus for the electrochemical reduction of an aqueous solution of carbonates, bicarbonates and/or CO₂ to alcohols and carboxylic acids.

### Examples of carrying out the Invention

In the following examples, product yields are given in % of faradaic efficiency unless otherwise indicated.

### Example 1: Apparatus for preparation of the starting electrolyte

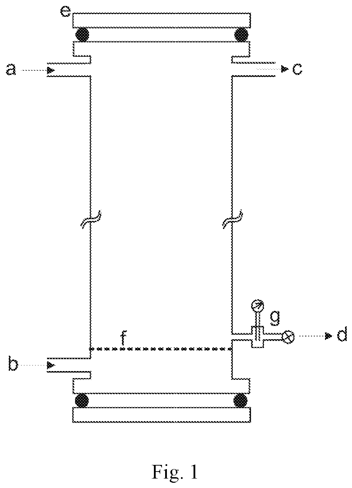

An example of the apparatus is shown in Fig. 1. The apparatus comprises a cylindrical body containing an inlet b at the bottom for the supply of CO₂-containing gas. Above the inlet b is a frit f for filtering off solid impurities from the gas. At the top of the cylindrical body is an inlet a for the inflow of an alkali metal hydroxide or ammonium hydroxide solution and an outlet c for gaseous substances. The outlet c may be fitted with a gas analyzer (not shown). There is also an outlet d above the frit f for the discharge of the prepared electrolyte, provided with a valve g, which may optionally be fitted with a pH meter. The apparatus is closed on the top and bottom by flanges e.

### Example 2: Apparatus for electrochemical reduction of aqueous solutions of carbonates, bicarbonates and/or CO₂ to alcohols and carboxylic acids

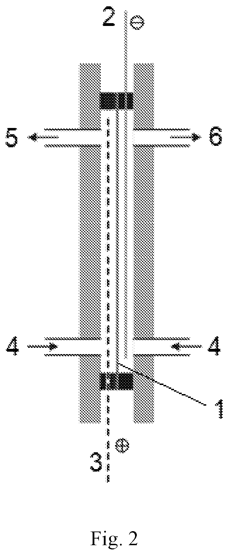

An example of the apparatus is shown in Fig. 2. The apparatus comprises a cylindrical body in which the cathode and anode compartments are separated by a proton exchange membrane (PEM) 1. A cathode 2 of copper silicide or mixed germanide/copper silicide is provided in the cathode compartment and an anode 3 is provided in the anode compartment. In further examples, a platinum or stainless-steel electrode was used as the anode 3. In the anode compartment, the cylindrical body is provided with an electrolyte inlet 4 and an anolyte outlet 5. In the cathode compartment, the cylindrical body is provided with an electrolyte inlet 4 and a catholyte outlet 6. The catholyte contains the electrochemical reduction products (alcohols, salts of carboxylic acids).

### Example 3: Apparatus for electrochemical transformation of carboxylic acid salts to carboxylic acid salts with a higher number of carbons

The apparatus shown in Fig. 2 may be used for electrochemical transformation of carboxylic acid salts to carboxylic acid salts with a higher number of carbons. In this embodiment, the cathode 2 is a copper germanide or a mixed copper germanide/silicide electrode.

### Example 4: Preparation of an electrolyte in the form of an aqueous solution of carbonates, bicarbonates and CO₂

The electrolyte was prepared in the apparatus described in Fig. 1. A CO₂ cylinder (13 % v/v, 200 atm) was used as CO₂ source in practical testing of the invention. The gaseous CO₂ was introduced into a 4M KOH solution. Carbonating 1 L of the solution resulted in an approximately 4M solution (KHCO₃, K₂CO₃) of CO₂ at a pH of approximately 6.9. The solution can be further diluted to the desired concentrations used in the examples below.

### Example 5: Preparation of an electrolyte in the form of an aqueous solution of C2-C5 salts of carboxylic acids

Saturated solutions of potassium acetate, potassium propionate or potassium valerate were prepared by neutralizing the corresponding acids with saturated KOH solution under constant stirring and heat removal.

### Example 6:

Electrochemical reduction of 1M aqueous solution of K₂CO₃ saturated with CO₂ to pH 6.8 was carried out in the apparatus shown in Fig. 2. The working voltage between the electrodes (copper silicide - cathode, Pt - anode) was set to 3.0 V. A current of about 100 mA (current density about 33 mA/cm²) flowed between the electrodes through the proton exchange membrane (PEM). The operating temperature was 23 °C. The volume of the cathodic compartment was about 3 ml. The electrolysis was carried out over 24 hours. After this time, the resulting mixture from the cathodic compartment was analyzed by magnetic resonance (NMR) and liquid chromatography with mass spectrometric detection (LC-MS). The liquid products included ethanol (82 % faradaic efficiency), 8 % potassium acetate, <2 % methanol, and the remainder were acetone, propanol, potassium formate. Hydrogen (<2 % faradaic efficiency) was identified as the main gaseous product from the cathodic compartment.

### Example 7:

Electrochemical reduction of 4M aqueous solution of potassium hydroxide saturated with CO₂ to pH ca 6.9 was carried out in the apparatus shown in Fig. 2. The working voltage between the electrodes (copper silicide - cathode, Pt - anode) was set to 2.8 V. A current of about 70 mA (current density about 25 mA/cm²) flowed between the electrodes through the PEM membrane. The operating temperature was 22 °C. The volume of the cathodic compartment was about 3 ml. The electrolysis was carried out for 168 hours. After this time, the resulting mixture from the cathodic compartment was analyzed by NMR and LC-MS. The liquid products included ethanol (75 % faradaic efficiency), 12 % potassium acetate, <3 % methanol, <2 % potassium propionate, about 2 % potassium valerate, the remainder was acetone, propanol, potassium formate. Hydrogen was identified as the main gaseous product from the cathodic compartment (<3 % faradaic efficiency).

### Example 8:

Electrochemical reduction of 1M aqueous solution of K₂CO₃ saturated with CO₂ to pH ca 6.9 was carried out in a device analogous to Fig. 2 without PEM membrane and with a cell volume of 50 ml with a reference electrode (Ag/AgCl (3.5 M KCl)). The working voltage between the electrodes (copper silicide - cathode, Pt - anode vs. reference electrode) was set to -1.5 V. The current flowing between the electrodes was about 50 mA (current density about 15 mA/cm²). The operating temperature was 23 °C. The electrolyte volume was about 40 ml. The electrolysis was carried out for 72 hours. After this time, the resulting mixture from the cathodic compartment was analyzed by NMR and LC-MS. The liquid products included ethanol (70% faradaic efficiency), 10% potassium acetate, <5% methanol, the remainder was acetone, propanol, potassium lactate, potassium formate, potassium propionate. Hydrogen was identified as the main gaseous product of water reduction (<2 % faradaic efficiency).

### Example 9:

Electrochemical reduction of 1M aqueous solution of K₂CO₃ saturated with CO₂ to pH 6.9 was carried out in the apparatus shown in Fig. 2. The working voltage between the electrodes (copper germanide - cathode, Pt - anode) was set to about 3.6 V. A current of approx. 250 mA (current density approx. 70 mA/cm²) flowed between the electrodes through the PEM membrane. The operating temperature was 23 °C. The volume of the cathodic compartment was about 3 ml. The electrolysis was carried out for 24 hours. After this time, the resulting mixture from the cathodic compartment was analyzed by NMR and LC-MS. The liquid products included methanol (25% faradaic efficiency), 20% potassium acetate, 20% ethanol, the remainder was acetone, propanol, potassium formate. Only hydrogen (<25 % faradaic efficiency) was identified as the main gaseous product from the cathodic compartment.

### Example 10:

Electrochemical reduction of 1M aqueous solution of potassium hydroxide saturated with CO₂ to pH 6.9 was carried out in the apparatus shown in Fig. 2. The working voltage between the electrodes (copper germanide - cathode, Pt - anode) was set to about 3.5 V. A current of approx. 350 mA (current density approx. 70 mA/cm²) flowed between the electrodes through the PEM membrane. The operating temperature was 22 °C. The volume of the cathodic compartment was about 3 ml. The electrolyte flow rate was tested between 0.001 and 2 per min. However, the embodiments of the invention are not limited thereby. The electrolysis was carried out for 48 hours. After this time, the resulting mixture from the cathodic compartment was analyzed by NMR and LC-MS. The liquid products included methanol (25% faradaic efficiency), 20% potassium acetate, 20% ethanol, and the remainder were acetone, propanol, potassium formate. Only hydrogen (<25 % faradaic efficiency) was identified as the main gaseous product from the cathodic compartment.

### Example 11:

Electrochemical reduction of 1M aqueous solution of K₂CO₃ at pH 11.4 ± 0.2 was carried out in the apparatus shown in Fig. 2. The working voltage between the electrodes (copper silicide - cathode, Pt - anode) was set to about 2.4 V. A current of approx. 60 mA (current density approx. 20 mA/cm²) flowed between the electrodes through the PEM membrane. The operating temperature was 23 °C. The volume of the cathodic compartment was about 3 ml. The electrolysis was carried out for 24 hours. After this time, the resulting mixture from the cathodic compartment was analyzed by NMR and LC-MS. The liquid products included potassium acetate (82 % faradaic efficiency), 8 % ethanol, < 5 % methanol, the remainder was acetone, propanol, potassium formate. Only hydrogen (<3 % faradaic efficiency) was identified as the major gaseous product from the cathodic compartment.

### Example 12:

Electrochemical reduction of 1M aqueous solution of K₂CO₃ at pH 11.4 ± 0.1 was carried out in the apparatus shown in Fig. 2. The working voltage between the electrodes (copper silicide/germanide - cathode, Pt - anode) was set to about 2.6 V. A current of approx. 80 mA (current density approx. 30 mA/cm²) flowed between the electrodes through the PEM membrane. The operating temperature was 22 °C. The volume of the cathodic compartment was about 3 ml. The electrolysis was carried out for 72 hours. After this time, the resulting mixture from the cathodic compartment was analyzed by NMR and LC-MS. The liquid products included methanol (30% faradaic efficiency), 25% potassium formate, <5% ethanol, the remainder was acetone, propanol. Only hydrogen (<8 % faradaic efficiency) was identified as the main gaseous product from the cathodic compartment.

### Example 13:

Electrochemical reduction of 1M aqueous solution of K₂CO₃ at pH 11.0 ± 0.2 was carried out in the apparatus shown in Fig. 2. The working voltage between the electrodes (copper silicide/germanide - cathode, Pt - anode) was set to about 3.6 V. A current of approx. 200 mA (current density approx. 60 mA/cm²) flowed between the electrodes through the PEM membrane. The operating temperature was 78 °C. The volume of the cathodic compartment was about 3 ml. The electrolysis was carried out for 72 hours. After this time, the resulting mixture from the cathodic compartment was analyzed by NMR and LC-MS. The liquid products included methanol (20% faradaic efficiency), 35% potassium formate, <3% ethanol, the remainder was acetone, propanol. Only hydrogen (<15 % faradaic efficiency) was identified as the main gaseous product from the cathodic compartment.

### Example 14:

Electrochemical transformation of a saturated potassium acetate solution at pH 11.0 ± 0.2 was carried out in the apparatus shown in Fig. 2. The working voltage between the electrodes (copper germanide - cathode, stainless steel mesh - anode) was set to about 3.6 V. A current of approx. 500 mA (current density approx. 350 mA/cm²) flowed between the electrodes. The operating temperature was 23 °C. The volume of the cathodic compartment was about 3 ml. The electrolysis was carried out for 144 hours. After this time, the resulting mixture from the cathodic compartment was analyzed by NMR and LC-MS. The liquid products included methanol (30% faradaic efficiency), 30% potassium propionate, <15% ethanol, and the remainder were acetone, propanol and potassium formate. Only hydrogen (<10 % faradaic efficiency) was identified as the major gaseous product from the cathodic compartment.

### Example 15:

Electrochemical transformation of a solution of potassium propionate at pH 11.5± 0.2 was carried out in the apparatus shown in Fig. 2. The working voltage between the electrodes (copper germanide - cathode, stainless steel mesh - anode) was set to about 3.8 V. The current flowing between the electrodes was about 110 mA (current density about 52 mA/cm²). The operating temperature was 23 °C. The electrolyte volume was about 3 ml. The electrolysis was carried out for 168 hours. After this time, the resulting mixture from the cathodic compartment was analyzed by NMR and LC-MS. The liquid products included methanol (25 % faradaic efficiency), 15 % potassium acetate, <10 % ethanol, 15 % potassium valerate, and the remainder was acetone, propanol and potassium formate. Only hydrogen (<15 % faradaic efficiency) was identified as the major gaseous product from the cathodic compartment.

### Example 16:

Electrochemical transformation of a potassium propionate solution at pH 11.3 ± 0.2 was carried out in the apparatus shown in Fig. 2. The working voltage between the electrodes (copper germanide - cathode, Pt - anode) was set to about 4.7-5.0 V. The current flowing between the electrodes was about 2.2 A (current density about 1100 mA/cm²). The operating temperature was about 81 °C. The electrolyte volume was about 3 ml. The electrolysis was carried out for 120 hours. After this time, the resulting mixture from the cathodic compartment was analyzed by NMR and LC-MS. The liquid products included methanol (15% faradaic efficiency), 12% potassium acetate, <12% ethanol, 10% potassium valerate, and the remainder was acetone, propanol and potassium formate. Only hydrogen (<35 % faradaic efficiency) was identified as the major gaseous product from the cathodic compartment.

### Example 17:

Electrochemical transformation of a potassium valerate solution at pH 11.5 ± 0.3 was carried out in the apparatus shown in Fig. 2. The working voltage between the electrodes (copper germanide - cathode, Pt - anode) was set to about 3.8-3.9 V. The current flowing between the electrodes was about 1.2 A (current density about 530 mA/cm²). The operating temperature was about 81 °C. The electrolyte volume was about 3 ml. The electrolysis was carried out for 72 hours. After this time, the resulting mixture from the cathodic compartment was analyzed by NMR and LC-MS. The liquid products included methanol (15% faradaic efficiency), 12% potassium acetate, <12% ethanol, 10% potassium nonanoate, and the remainder was acetone, propanol and potassium formate. Hydrogen (<20 % faradaic efficiency) was identified as the major gaseous product from the cathodic compartment.

### Example 18:

Electrochemical reduction of 1M aqueous Na₂CO₃ solution at pH 11.0 ± 0.2 was carried out in the apparatus shown in Fig. 2. The working voltage between the electrodes (copper silicide - cathode, stainless steel mesh - anode) was set to about 2.4 V. A current of approx. 60 mA (current density approx. 20 mA/cm²) flowed between the electrodes through the PEM membrane. The operating temperature was 23 °C. The volume of the cathodic compartment was about 3 ml. The electrolysis was carried out for 24 hours. After this time, the resulting mixture from the cathodic compartment was analyzed by NMR and LC-MS. The liquid products included sodium acetate (60 % faradaic efficiency), 5 % ethanol, < 4 % methanol, the remainder was acetone, sodium formate. Only hydrogen (<20 % faradaic efficiency) was identified as the main gaseous product from the cathodic compartment.

### Example 19:

Electrochemical reduction of 1M aqueous solution of Li₂CO₃ at pH 10.9 ± 0.1 was carried out in the apparatus shown in Fig. 2. The working voltage between the electrodes (copper silicide - cathode, stainless steel mesh - anode) was set to about 2.2 V. A current of approx. 40 mA (current density approx. 15 mA/cm²) flowed between the electrodes through the PEM membrane. The operating temperature was 23 °C. The volume of the cathodic compartment was about 3 ml. The electrolysis was carried out for 24 hours. After this time, the resulting mixture from the cathodic compartment was analyzed by NMR and LC-MS. The liquid products included lithium acetate (55 % faradaic efficiency), <10 % methanol, the remainder was lithium formate. Only hydrogen (<25 % faradaic efficiency) was identified as the main gaseous product from the cathodic compartment.

### Example 20:

Electrochemical reduction of 1M aqueous solution of Cs₂CO₃ at pH 11.1± 0.2 was carried out in the apparatus shown in Fig. 2. The working voltage between the electrodes (copper silicide - cathode, platinum - anode) was set to about 2.4 V. A current of approx. 65 mA (current density approx. 22 mA/cm²) flowed between the electrodes through the PEM membrane. The operating temperature was 23 °C. The volume of the cathodic compartment was about 3 ml. The electrolysis was carried out for 48 hours. After this time, the resulting mixture from the cathodic compartment was analyzed by NMR and LC-MS. The liquid products included caesium acetate (85% faradaic efficiency), 9% ethanol, <2% methanol, the remainder was acetone, propanol, caesium formate. Only hydrogen (<2 % faradaic efficiency) was identified as the main gaseous product from the cathodic compartment.

### Example 21:

Electrochemical reduction of a mixed 1M aqueous solution of K₂CO₃ with 1 M aqueous solution of (NH₄)₂CO₃ (in the ratio of solutions 3:1 v/v) at pH 10.5 ± 0.3 was carried out in the apparatus shown in Fig. 2. The working voltage between the electrodes (copper silicide - cathode, Pt - anode) was set to about 3.0 V. A current of approx. 500 mA (current density approx. 150 mA/cm²) flowed between the electrodes through the PEM membrane. The operating temperature was 40 °C. The volume of the cathodic compartment was about 3 ml. The electrolysis was carried out for 72 hours. After this time, the resulting mixture from the cathodic compartment was analyzed by NMR and LC-MS. The liquid products included potassium/ammonium acetate (>90 % faradaic efficiency), 3 % potassium/ammonium formate, <3 % ethanol, the remainder was acetone, propanol. Only hydrogen was identified as the main gaseous product from the cathodic compartment (<3 % faradaic efficiency).

### Example 22:

Electrochemical reduction of a saturated aqueous solution of (NH₄)₂CO₃ at pH 8.9 ± 0.3 was carried out in the apparatus shown in Fig. 2. The working voltage between the electrodes (copper silicide - cathode, Pt - anode) was set to about 5.0 V. A current of approx. 1500 mA (current density approx. 600 mA/cm²) flowed between the electrodes through the PEM membrane. The operating temperature was 50 °C. The volume of the cathodic compartment was about 3 ml. The electrolysis was carried out for 72 hours and a saturated ammonium carbonate solution was added. After this time, the resulting mixture from the cathodic compartment was analyzed by NMR and LC-MS. The liquid products included potassium/ammonium acetate (>95% faradaic efficiency), <1% potassium/ammonium formate, <1% ethanol, the remainder being acetone, propanol. Only hydrogen (<1 % faradaic efficiency) was identified as the main gaseous product from the cathodic compartment, together with ammonia.

### Example 23:

Electrochemical reduction of a mixture of 1 M aqueous solution of NH₄H₂PO₄ and 1 M aqueous solution of (NH₄)₂CO₃ (3:1 V/V ratio) at pH 6.6 ± 0.2 was carried out in the apparatus shown in Fig. 2. The working voltage between the electrodes (copper silicide - cathode, Pt - anode) was set to about 3.0 V. A current of approx. 100 mA (current density approx. 30 mA/cm²) flowed between the electrodes through the PEM membrane. The operating temperature was 40 °C. The volume of the cathodic compartment was about 50 ml. The electrolysis was carried out for 144 hours and a saturated ammonium carbonate solution was added. After this time, the resulting mixture from the cathodic compartment was analyzed by NMR and LC-MS. The liquid products included ammonium acetate (>60% faradaic efficiency), <20% ethanol, <5% ammonium formate, the remainder was acetone, propanol. Only hydrogen (<5 % faradaic efficiency) was identified as the main gaseous product from the cathodic compartment, together with ammonia.

### Example 24:

The electrochemical reduction of a mixture of 4M aqueous solution of K₂CO₃ and saturated aqueous solution of (NH₄)₂CO₃ (1:2 V/V ratio) at pH 10.9 ± 0.4 was carried out in the apparatus shown in Fig. 2. The working voltage between the electrodes (copper silicide - cathode, Pt - anode) was set to about 5.0 V. A current of approx. 900 mA (current density approx. 300 mA/cm²) flowed between the electrodes through the PEM membrane. The operating temperature was 50 °C. The volume of the cathodic compartment was about 100 ml. The electrolysis was carried out for 72 hours. After this time, the resulting mixture from the cathodic compartment was analyzed by NMR and LC-MS. The liquid products included potassium/ammonium acetate (>91 % faradaic efficiency), <1 % potassium/ammonium formate, <1 % ethanol, the remainder being acetone, propanol, ethylene glycol. Only hydrogen was identified as the main gaseous product from the cathodic compartment (<5 % faradaic efficiency).

### Example 25:

The electrochemical reduction of a mixture of 0.1 M aqueous solution of K₂CO₃ and 0.1 M aqueous solution of (NH₄)₂CO₃ (1:4 V/V ratio) at pH 9.5 ± 0.2 was carried out in the apparatus shown in Fig. 2. The working voltage between the electrodes (copper silicide - cathode, Pt - anode) was set to about 3.0 V. A current of approx. 100 mA (current density approx. 30 mA/cm²) flowed between the electrodes through the PEM membrane. The operating temperature was 30 °C. The volume of the cathodic compartment was about 3 ml. The electrolysis was carried out for 24 hours. After this time, the resulting mixture from the cathodic compartment was analyzed by NMR and LC-MS. The liquid products included potassium/ammonium acetate (>90 % faradaic efficiency), <2 % potassium/ammonium formate, <1 % ethanol, the remainder being acetone, propanol, ethylene glycol, oxalic acid). Only hydrogen was identified as the major gaseous product from the cathodic compartment (<5 % faradaic efficiency). Ammonia was further identified in the gas phase.

### Example 26:

Electrochemical reduction of 0.1M aqueous solution of K₂CO₃ at pH 9.7 ± 0.2 was carried out in the apparatus shown in Fig. 2. The working voltage between the electrodes (copper silicide - cathode, Pt - anode) was set to about 3.0 V. Ammonia gas saturated with water (ammonia-water solution) was added to the electrolyte in the cathodic part of the electrolyzer. A current of about 100 mA (current density about 30 mA/cm²) flowed between the electrodes through the PEM membrane. The operating temperature was 30 °C. The volume of the cathodic compartment was about 3 ml. The electrolysis was carried out for 24 hours. After this time, the resulting mixture from the cathodic compartment was analyzed by NMR and LC-MS. The liquid products included potassium/ammonium acetate (>90 % faradaic efficiency), <2 % potassium/ammonium formate, <1 % ethanol, the rest being acetone, propanol, ethylene glycol, oxalic acid. Only hydrogen was identified as the major gaseous product from the cathodic compartment (<5 % faradaic efficiency). Ammonia was further identified in the gas phase.

### Example 27:

Electrochemical reduction of concentrated acetic acid with traces of water at pH <1 ± 0.4 was carried out in the apparatus of Fig. 2. The working voltage between the electrodes (copper silicide - cathode, Pt - anode) was set to about 3.0 V. A current of approx. 100 mA (current density approx. 30 mA/cm²) flowed between the electrodes through the PEM membrane. The operating temperature was 60 °C. The volume of the cathodic compartment was about 10 ml. The electrolysis was carried out for 144 hours. After this time, the resulting mixture from the cathodic compartment was analyzed by NMR and LC-MS. The liquid products included acetic acid methyl ester (>93% faradaic efficiency), <1% dimethyl carbonate, <1% ethanol, the remainder being traces of acetone, propanol, ethylene glycol. Only hydrogen (<5 % faradaic efficiency) was identified as the major gaseous product from the cathodic compartment.

## Claims

1. Method for electrochemically converting a starting electrolyte, selected from aqueous solutions of alkali metal carbonate, ammonium carbonate, alkali metal bicarbonate, ammonium bicarbonate, CO₂, a C2-C5 carboxylic acid salt with an alkali metal or ammonium, or mixtures thereof, optionally a mixture thereof with ammonia, to form alcohols and/or carboxylic acid salts, **characterized in that** the said method is carried out in an electrochemical reactor in which the cathode is a catalyst comprising copper silicide, copper germanide and/or mixed copper germanide/silicide.

2. The method according to claim 1, wherein the electrochemical reactor comprises a cathode compartment and an anode compartment, and these compartments are separated by a proton exchange membrane.

3. The method according to claim 1 or 2, wherein the starting electrolyte is selected from aqueous solutions of alkali metal carbonate, ammonium carbonate, alkali metal bicarbonate, ammonium bicarbonate, CO₂ or a mixture thereof, optionally a mixture thereof with ammonia, wherein the reaction produces alcohols and salts of carboxylic acids, and wherein the cathode is a copper silicide and/or mixed germanide/copper silicide cathode.

4. The method according to claim 3, wherein pH value of the starting electrolyte is between 6.7 and 9.

5. The method of claim 3, wherein pH value of the starting electrolyte is greater than 9.

6. The method according to claim 1 or 2, wherein the starting electrolyte is a solution of a C2-C5 carboxylic acid salt with an alkali metal or ammonium, wherein the reaction produces alcohols and alkali metal or ammonium salt(s) of carboxylic acid(s) having a higher carbon number than the starting carboxylic acid, and wherein the cathode is a copper germanide and/or a mixed copper germanide/silicide cathode.

7. The method according to claim 1 or 2, wherein the starting electrolyte is a solution of a C2-C5 carboxylic acid, wherein the reaction produces an alcohol which may optionally further react in situ with the carboxylic acid to form an ester, and wherein the cathode is a copper silicide and/or mixed copper germanide/silicide cathode.

8. The method according to claim 1 or 2, wherein the starting electrolyte is selected from aqueous solutions of alkali metal carbonate, ammonium carbonate, alkali metal bicarbonate, ammonium bicarbonate, CO₂ or a mixture thereof, optionally a mixture thereof with ammonia, optionally further containing a salt of C2-C5 carboxylic acid and alkali metal or ammonium, wherein salts of lower carboxylic acids, typically formate and acetate, are formed first, followed by a second in situ reaction of the C2-C5 carboxylic acid salts to carboxylic acid salts having more carbons than the number of carbons of the starting C2-C5 carboxylic acids, and wherein the cathode is a cathode made of mixture of copper silicide and copper germanide or a mixed germanide/copper silicide cathode.

9. The method according to any one of the preceding claims, wherein the starting electrolyte contains ammonium cations.

10. The method according to any one of the preceding claims, wherein the starting electrolyte contains ammonium carbonate, and optionally also ammonia.

11. The method according to any one of the preceding claims, **characterized in that** the electrochemical reactor contains a platinum anode or stainless-steel anode.

12. The method according to any one of claims 1 to 5 and 8, wherein the starting electrolyte, which is a solution selected from the solutions of alkali metal carbonate, ammonium carbonate, alkali metal bicarbonate, ammonium bicarbonate, CO₂ or a mixture thereof, optionally a mixture thereof with ammonia, is prepared by first preparing a saturated alkali metal hydroxide aqueous solution which is then saturated with a gas containing CO₂, NH₃, preferably to a pH of not lower than 6.7.

13. The method according to any one of claims 1, 2 or 6, wherein the starting electrolyte, which is a solution of an alkali metal salt of C2-C5 carboxylic acid or ammonium salt of C2-C5 carboxylic acid, is prepared by adding a saturated solution of alkali metal hydroxide or ammonium hydroxide to a solution of the corresponding acid, completely neutralizing the acid.

## Patentansprüche

1. Verfahren zur elektrochemischen Umwandlung eines Ausgangselektrolyten, ausgewählt aus wässrigen Lösungen von Alkalimetallcarbonat, Ammoniumcarbonat, Alkalimetallhydrogencarbonat, Ammoniumhydrogencarbonat, CO₂, einem C2-C5-Carbonsäuresalz mit einem Alkalimetall oder Ammonium oder Mischungen davon, gegebenenfalls einer Mischung davon mit Ammoniak, zu Alkoholen und/oder Carbonsäuresalzen, **dadurch gekennzeichnet, dass** das Verfahren in einem elektrochemischen Reaktor durchgeführt wird, dessen Kathode ein Katalysator ist, der Kupfersilicid, Kupfergermanid und/oder ein Gemisch von Kupfergermanid und -silicid enthält.

2. Verfahren nach Anspruch 1, wobei der elektrochemische Reaktor ein Kathodenkompartiment und ein Anodenkompartiment umfasst, die durch eine Protonenaustauschmembran getrennt sind.

3. Verfahren nach Anspruch 1 oder 2, wobei der Ausgangselektrolyt ausgewählt ist aus wässrigen Lösungen von Alkalimetallcarbonat, Ammoniumcarbonat, Alkalimetallhydrogencarbonat, Ammoniumhydrogencarbonat, CO₂ oder einem Gemisch davon, gegebenenfalls einem Gemisch davon mit Ammoniak, wobei die Reaktion Alkohole und Salze von Carbonsäuren erzeugt und wobei die Kathode eine Kupfersilicid- und/oder eine gemischte Kupfergermanid/Silicid-Kathode ist.

4. Verfahren nach Anspruch 3, wobei der pH-Wert des Ausgangselektrolyten zwischen 6,7 und 9 liegt.

5. Verfahren nach Anspruch 3, wobei der pH-Wert des Ausgangselektrolyten größer als 9 ist.

6. Verfahren nach Anspruch 1 oder 2, wobei der Ausgangselektrolyt eine Lösung eines C2-C5-Carbonsäuresalzes mit einem Alkalimetall oder Ammonium ist, wobei die Reaktion Alkohole und Alkalimetall- oder Ammoniumsalze von Carbonsäuren mit einer höheren Kohlenstoffzahl als die Ausgangscarbonsäure erzeugt, und wobei die Kathode eine Kupfergermanid- und/oder eine gemischte Kupfergermanid/Silicid-Kathode ist.

7. Verfahren nach Anspruch 1 oder 2, wobei der Ausgangselektrolyt eine Lösung einer C2-C5-Carbonsäure ist, wobei die Reaktion einen Alkohol erzeugt, der gegebenenfalls in situ mit der Carbonsäure zu einem Ester weiterreagiert, und wobei die Kathode eine Kupfersilicid- und/oder eine gemischte Kupfergermanid/Silicid-Kathode ist.

8. Verfahren nach Anspruch 1 oder 2, wobei der Ausgangselektrolyt ausgewählt ist aus wässrigen Lösungen von Alkalimetallcarbonat, Ammoniumcarbonat, Alkalimetallhydrogencarbonat, Ammoniumhydrogencarbonat, CO₂ oder einem Gemisch davon, gegebenenfalls einem Gemisch davon mit Ammoniak, gegebenenfalls zusätzlich ein Salz einer C2-C5-Carbonsäure und eines Alkalimetalls oder Ammoniums enthält, wobei zunächst Salze niederer Carbonsäuren, typischerweise Formiat und Acetat, gebildet werden, gefolgt von einer zweiten In-situ-Reaktion der C2-C5-Carbonsäuresalze zu Carbonsäuresalzen mit mehr Kohlenstoffatomen als die Ausgangscarbonsäuren, und wobei die Kathode aus einem Gemisch von Kupfersilicid und Kupfergermanid oder aus einer Mischung von Kupfergermanid/Kupfersilicid besteht.

9. Verfahren nach einem der vorhergehenden Ansprüche, wobei der Ausgangselektrolyt Ammoniumkationen enthält.

10. Verfahren nach einem der vorhergehenden Ansprüche, wobei der Ausgangselektrolyt Ammoniumcarbonat und gegebenenfalls auch Ammoniak enthält.

11. Verfahren nach einem der vorhergehenden Ansprüche, **dadurch gekennzeichnet, dass** der elektrochemische Reaktor eine Platin- oder Edelstahlanode enthält.

12. Verfahren nach einem der Ansprüche 1 bis 5 und 8, wobei der Ausgangselektrolyt, eine Lösung von Alkalimetallcarbonat, Ammoniumcarbonat, Alkalimetallhydrogencarbonat, Ammoniumhydrogencarbonat, CO₂ oder einem Gemisch davon, gegebenenfalls mit Ammoniak, hergestellt wird, indem zunächst eine gesättigte wässrige Alkalimetallhydroxidlösung hergestellt und anschließend mit einem CO₂ und/oder NH₃ enthaltenden Gas, vorzugsweise auf einen pH-Wert von mindestens 6,7, gesättigt wird.

13. Verfahren nach einem der Ansprüche 1, 2 oder 6, wobei der Ausgangselektrolyt, eine Lösung eines Alkalimetallsalzes einer C2-C5-Carbonsäure oder eines Ammoniumsalzes einer C2-C5-Carbonsäure, durch Zugabe einer gesättigten Alkalimetallhydroxid- oder Ammoniumhydroxidlösung zu einer Lösung der entsprechenden Säure unter vollständiger Neutralisation der Säure hergestellt wird.

## Revendications

1. Procédé de conversion électrochimique d'un électrolyte initial, choisi parmi des solutions aqueuses de carbonate de métal alcalin, de carbonate d'ammonium, de bicarbonate de métal alcalin, de bicarbonate d'ammonium, de CO₂, d'un sel d'acide carboxylique en C2-C5 avec un métal alcalin ou avec l'ammonium, ou de mélanges de ces solutions, éventuellement avec de l'ammoniac, pour former des alcools et/ou des sels d'acide carboxylique, le procédé étant **caractérisé en ce qu'**il est mis en œuvre dans un réacteur électrochimique dont la cathode est un catalyseur comprenant du siliciure de cuivre, du germanure de cuivre et/ou un mélange de germanure et de siliciure de cuivre.

2. Procédé selon la revendication 1, dans lequel le réacteur électrochimique comprend un compartiment cathodique et un compartiment anodique, séparés par une membrane échangeuse de protons.

3. Procédé selon la revendication 1 ou 2, dans lequel l'électrolyte initial est choisi parmi des solutions aqueuses de carbonate de métal alcalin, de carbonate d'ammonium, de bicarbonate de métal alcalin, de bicarbonate d'ammonium, de CO₂ ou un mélange de ceux-ci, éventuellement un mélange de ceux-ci avec de l'ammoniac, la réaction produisant des alcools et des sels d'acides carboxyliques, et la cathode étant une cathode en siliciure de cuivre et/ou une cathode mixte en germanure/siliciure de cuivre.

4. Procédé selon la revendication 3, dans lequel le pH de l'électrolyte initial est compris entre 6,7 et 9.

5. Procédé selon la revendication 3, dans lequel le pH de l'électrolyte initial est supérieur à 9.

6. Procédé selon la revendication 1 ou 2, dans lequel l'électrolyte initial est une solution d'un sel d'acide carboxylique en C2-C5 avec un métal alcalin ou avec l'ammonium, la réaction produisant des alcools et un ou plusieurs sels de métal alcalin ou d'ammonium et d'acide(s) carboxylique(s) ayant un nombre d'atomes de carbone supérieur à celui de l'acide carboxylique initial, et dans lequel la cathode est une cathode en germanure de cuivre et/ou une cathode mixte en germanure/siliciure de cuivre.

7. Procédé selon la revendication 1 ou 2, dans lequel l'électrolyte initial est une solution d'un acide carboxylique en C2-C5, la réaction produisant un alcool qui peut éventuellement réagir in situ avec l'acide carboxylique pour former un ester, et dans lequel la cathode est une cathode en siliciure de cuivre et/ou une cathode mixte en germanure/siliciure de cuivre.

8. Procédé selon la revendication 1 ou 2, dans lequel l'électrolyte initial est choisi parmi des solutions aqueuses de carbonate de métal alcalin, de carbonate d'ammonium, de bicarbonate de métal alcalin, de bicarbonate d'ammonium, de CO₂ ou un mélange de ceux-ci, éventuellement un mélange de ceux-ci avec de l'ammoniac, et contenant éventuellement en outre un sel d'acide carboxylique en C2-C5 et de métal alcalin ou d'ammonium, et dans lequel les sels d'acides carboxyliques inférieurs, typiquement le formiate et l'acétate, sont d'abord formés, ce qui est suivi d'une seconde réaction in situ d'acide carboxylique en C2-C5 pour former des sels d'acides carboxyliques comportant plus d'atomes de carbone que les acides carboxyliques en C2-C5 initiaux, et dans lequel la cathode est constituée d'un mélange de siliciure de cuivre et de germanure de cuivre ou la cathode est une cathode mixte en germanure/siliciure de cuivre.

9. Procédé selon l'une quelconque des revendications précédentes, dans lequel l'électrolyte initial contient des cations ammonium.

10. Procédé selon l'une quelconque des revendications précédentes, dans lequel l'électrolyte initial contient du carbonate d'ammonium et, éventuellement, de l'ammoniac.

11. Procédé selon l'une quelconque des revendications précédentes, **caractérisé en ce que** le réacteur électrochimique contient une anode en platine ou en acier inoxydable.

12. Procédé selon l'une quelconque des revendications 1 à 5 et 8, dans lequel l'électrolyte initial, qui est une solution choisie parmi les solutions de carbonate de métal alcalin, de carbonate d'ammonium, de bicarbonate de métal alcalin, de bicarbonate d'ammonium, de CO₂ ou un mélange de ceux-ci, éventuellement un mélange de ceux-ci avec de l'ammoniac, est préparé en préparant d'abord une solution aqueuse saturée d'hydroxyde de métal alcalin, puis en la saturéant avec un gaz contenant du CO₂ ou du NH₃, de préférence jusqu'à un pH pas inférieur à 6,7.

13. Procédé selon l'une quelconque des revendications 1, 2 ou 6, dans lequel l'électrolyte initial, qui est une solution d'un sel de métal alcalin d'acide carboxylique en C2-C5 ou d'un sel d'ammonium d'acide carboxylique en C2-C5, est préparé en ajoutant une solution saturée d'hydroxyde de métal alcalin ou d'hydroxyde d'ammonium à une solution de l'acide correspondant, neutralisant complètement l'acide.
